# EUROPÄISCHE PATENTSCHRIFT

(11) **EP 4 698 264 B1**
(45) Veröffentlichungstag und Bekanntmachung des Hinweises auf die Patenterteilung: **19.08.2026**
(21) Anmeldenummer: 25739521.0
(22) Anmeldetag: 01.07.2025
(51) Int. Cl.: A61M 37/00

(54) **VERFAHREN ZUR DURCHFÜHRUNG EINER KÖRPERMODIFIKATION UND VERBUNDBOGEN**
METHOD FOR CARRYING OUT A BODY MODIFICATION AND COMPOSITE SHEET
PROCÉDÉ POUR EFFECTUER UNE MODIFICATION CORPORELLE ET FEUILLE COMPOSITE

(30) Priorität: 08.07.2024 DE 102024119290
(43) Veröffentlichungstag der Anmeldung: 25.02.2026
(73) Patentinhaber: Trier, Philipp, 71334 Waiblingen (DE)
(72) Erfinder: Trier, Philipp, 71334 Waiblingen (DE)
(74) Vertreter: DREISS Patentanwälte PartG mbB
(86) Internationale Anmeldenummer: PCT/EP2025/068709
(87) Internationale Veröffentlichungsnummer: WO 2026/012838

(56) Entgegenhaltungen:
- EP-A2- 0 908 195
- WO-A1-2024/056990
- WO-A1-99/61234
- DE-A1- 102004 043 447
- US-A- 4 886 079
- US-A1- 2011 268 873
- US-A1- 2012 031 301
- US-A1- 2015 059 968

## Beschreibung

Die Erfindung betrifft ein Verfahren zur Durchführung einer Körpermodifikation mit Merkmalen des Anspruchs 1 und einen Verbundbogen mit Merkmalen des nebengeordneten Anspruchs.

In der Regel wird zum Tätowieren zunächst ein Tattoo-Design temporär auf die Haut übertragen. Das temporäre Übertragen des Tattoo-Designs auf die Haut erfolgt mittels eines speziellen Transferpapiers, welches das Tattoo-Design aufweist. Zunächst kann eine Transferflüssigkeit auf die zu tätowierende Körperstelle aufgebracht werden. Danach kann das Transferpapier, welches das Tattoo-Design umfasst, auf die Körperstelle aufgebracht und anschließend wieder entfernt werden. Dabei bleibt ein Abdruck des Tattoo-Designs vom Transferpapier auf der zu tätowierenden Körperstelle zurück. Dieser Abdruck kann als Schablone während des Tätowierens benutzt werden. Das eigentliche Tätowieren erfolgt daraufhin mittels eines Tätowierwerkzeugs, das mindestens eine Nadel umfasst. Dabei wird mittels des Tätowierwerkzeugs in die zu tätowierende Körperstelle eingestochen und (zeitgleich) Tinte injiziert.

Nachteilig dabei ist, dass der Abdruck leicht verzerren oder verschmieren kann, was zu Ungenauigkeiten beim Tätowieren führen kann.

EP 0 908 195 A2 offenbart ein kosmetisches Verfahren. DE 10 2004 043 447 A1 offenbart ein Tattoo-Appliziersystem. US 4 886 079 A offenbart ein kosmetisches Verfahren.

US 2011 / 268 873 A1 offenbart ein mit einem Tintenstrahldrucker gedrucktes Tattoo-Transfermuster. US 2015 / 059 968 A1 offenbart eine Vorrichtung und ein Verfahren zum Drucken von funktionellem Material auf biokompatiblem Dünnfilm. WO 99 / 61234 A1 offenbart von einem Benutzer erstellte temporäre Tattoos. US 2012 / 031 301 A1 offenbart eine Zusammensetzung einer Tätowierungsschablone und ein Verfahren zu ihrer Herstellung. WO 2024 / 056 990 A1 offenbart temporäre Tattoos. DE 10 2004 043 447 A1 offenbart ein temporäres Tattoo.

Die obige Aufgabe wird durch ein Verfahren zur Durchführung einer Körpermodifikation mit den Merkmalen des Anspruchs 1 gelöst. Das Verfahren umfasst die Schritte:
Anbringen (bzw. Aufbringen) einer Folie auf einen zu modifizierenden Körper. Die Folie weist mindestens eine Markierung zur Durchführung der Körpermodifikation auf.

Durchführen der Körpermodifikation am Körper entsprechend der Markierung. Die Folie wird während der Durchführung der Körpermodifikation an der Markierung, insbesondere mittels eines Körpermodifikationswerkzeugs, durchdrungen, um den Körper zu modifizieren. Es wird durch die Folie in den Körper eingestochen, insbesondere mittels des Körpermodifikationswerkzeugs, vorzugsweise eines TätowierWerkzeugs. Die Folie wird dabei durchstochen.

Hierdurch kann die Folie, insbesondere die Markierung, als Schablone auf dem Körper genutzt werden. Ein Verzerren oder Verschmieren der Markierung kann verhindert oder zumindest reduziert werden, wodurch die Genauigkeit, insbesondere beim Tätowieren, erhöht werden kann. Außerdem kann der Aufwand für die Durchführung der Körpermodifikation deutlich gesenkt werden.

Die Folie kann während der Durchführung der Körpermodifikation, insbesondere während der gesamten Dauer der Durchführung der Körpermodifikation, auf dem Körper verbleiben.

Bei dem Körpermodifikationswerkzeug kann es sich um ein Tätowierwerkzeug handeln, insbesondere wenn es sich bei der Körpermodifikation um eine Tätowierung handelt. Das Tätowierwerkzeug kann mindestens eine Nadel, insbesondere eine Tätowiernadel, umfassen. Mittels des Tätowierwerkzeugs, insbesondere mittels der Nadel, kann die Körpermodifikation (das Tätowieren), durchgeführt werden. Hierzu kann das Tätowierwerkzeug, insbesondere die Nadel, an der Markierung durch die Folie in den Körper, insbesondere in die Haut des Körpers, eingestochen werden, um Tätowierfarbe in den Körper, insbesondere in die Haut des Körpers, zu injizieren.

Dieser Vorgang kann so oft an der Markierung, insbesondere sukzessive an verschiedenen Stellen der Markierung, der Folie durchgeführt werden, bis die Tätowierung fertiggestellt ist. Mit anderen Worten kann dieser Vorgang entlang der Markierung so oft durchgeführt werden, bis die Tätowierung fertiggestellt ist. Die Folie kann an der Markierung oder entlang der Markierung mehrfach durchstochen und dabei in den Körper, insbesondere in die Haut, gestochen werden, um Tätowierfarbe in den Körper, insbesondere in die Haut des Körpers, zu injizieren.

Bei dem Körpermodifikationswerkzeug kann es sich ebenso um ein Piercingwerkzeug, insbesondere eine Piercingzange, handeln, insbesondere wenn es sich bei der Körpermodifikation um ein Piercing handelt.

Hierdurch kann auf eine Markierung einer Piercing-Sollstelle auf der Haut bspw. mittels eines Markers verzichtet werden.

Das Verfahren kann ein Einsetzen eines Schmucks in eine Öffnung in der Haut umfassen, die mittels des Piercingwerkzeugs, insbesondere der Piercingzange, erzeugt wurde.

Bei dem Körper kann es sich um eine Körperstelle, vorzugsweise eine äußere Körperstelle, oder ein Körperteil handeln. Bei dem Körper kann es sich um einen menschlichen oder tierischen Körper handeln. Bei dem menschlichen oder tierischen Körperteil kann es sich um einen Arm, einen Rücken, ein Bein, eine Brust, einen Bauch, usw. handeln. Das Durchführen der Körpermodifikation am menschlichen oder tierischen Körper kann auf und/oder in der Haut des Körpers, des Körperteils oder der Körperstelle erfolgen. Mit anderen Worten kann die Folie auf der Haut aufgebracht werden und insbesondere mittels des Körpermodifikationswerkzeugs durchstochen werden, um Tätowierfarbe in den Körper, insbesondere in die Haut des Körpers, zu injizieren.

Bei der Ausführung des Verfahrens können ausschließlich Mittel, Stoffe und/oder Werkstoffe zum Einsatz kommen, die biokompatibel, hyperallergen und/oder inert sind.

Unter biokompatiblen Stoffen können Stoffe verstanden werden, die in medizinischen, kosmetischen oder anderen Anwendungen genutzt werden können, ohne schädliche Auswirkungen auf den menschlichen Körper zu haben. Unter hyperallergenen Stoffen können Stoffe verstanden werden, die kein oder lediglich ein geringes Potenzial zur Auslösung von allergischen Reaktionen haben. Unter inerten Stoffen können Stoffe verstanden werden, die keine chemischen Reaktionen mit der Umgebung oder dem Körper eingehen. Unter den Mitteln, Stoffen und Werkstoffen, die während des Verfahrens zum Einsatz kommen, können die Folie, die Markierung, das Körpermodifikationswerkzeug, die Tätowierfarbe, die Reinigungslösung und/oder ein Sprühkleber (vgl. weiter unten) gemeint sein.

Bei der Körpermodifikation kann es sich um eine temporäre oder dauerhafte Körpermodifikation handeln.

Das Aufbringen der Folie kann derart erfolgen, dass die Folie sich flächig über den Körper oder einen Teil des Körpers erstreckt.

Die Folie kann organisch, biologisch abbaubar, vegan, ungefährlich, unbedenklich und/oder ohne Einschränkung verträglich ausgebildet sein.

Die Folie kann neu positioniert werden, wobei ein Reinigungs-Zwischenschritt, ein Geisterbild und eine Nachproduktion bei einer ersten falschen Positionierung vermieden werden können.

Gemäß einer Weiterbildung des Verfahrens kann die Markierung auf die Folie aufgedruckt werden, bevor die Folie auf dem Körper aufgebracht wird. Dies kann mittels eines Tintenstrahldruckers umgesetzt werden. Während des Aufdruckens kann die Folie mit einem Trägerpapier verbunden sein und Teil eines Verbundbogens sein (vgl. weiter unten).

Hierdurch kann die Markierung einfach, mit wenig Aufwand und kostengünstig auf die Folie aufgebracht werden.

Vorzugsweise kann es sich bei dem Tintenstrahldrucker um einen handelsüblichen Tintenstrahldrucker handeln. Hierdurch können die Kosten für die Durchführung des Verfahrens reduziert werden.

Es kann ein mobiler Einsatz ohne Notwendigkeit von produktgebundenen Spezialdruckern umgesetzt werden. Bei herkömmlichem Tätowieren wird üblicherweise ein Thermotransferverfahren verwendet, wobei ein Thermotransferdrucker oder Thermodruckers benötigt wird.

Gemäß einer Weiterbildung des Verfahrens kann das Anbringen (bzw. Aufbringen) der Folie mittels einer temporären Klebeverbindung, insbesondere mittels eines Sprühklebers, erfolgen. Dabei kann es sich um einen medizinischen Sprühkleber handeln.

Es ist ebenso denkbar, dass die Folie mittels eines Klebestreifens am Körper befestigt werden kann.

Hierdurch kann die Folie mit einfachen Mitteln angebracht werden. Es kann zudem sichergestellt werden, dass die Folie wieder vom Körper, bspw. durch ein Abziehen der Folie vom Körper, wieder entfernt werden kann, wenn das Durchführen der Körpermodifikation abgeschlossen ist. Die Markierung (bzw. das Konzept) kann hierdurch geschützt werden, da die Verklebung auf der dem Körper zugewandten Seite der Folie umgesetzt werden kann. So ist eine vollständige und/oder durchgehend randscharfe Darstellung der Markierung (bzw. des Konzepts) umsetzbar.

Es ist ein (nahezu) faltenfreies Verkleben der Folie an widrigen schwierigen und "unruhigen" Körperstellen wie z.B. Kniegelenk, Ellebogengelenk und Ähnlichem möglich.

Gemäß einer Weiterbildung des Verfahrens kann die Folie wasserlöslich, insbesondere kaltwasserlöslich, ausgebildet sein. Alternativ oder zusätzlich kann die Folie nach der Durchführung der Körpermodifikation mittels (kaltem) Wasser und/oder einer Reinigungslösung aufgelöst werden.

Das Verfahren kann den Schritt umfassen:
Auflösen der Folie mittels (kaltem) Wasser und/oder einer Reinigungslösung.

Hierdurch kann die Folie mit einfachen Mitteln einfach und vollständig vom Körper entfernt werden. Mit anderen Worten kann hierbei auf das Abziehen der Folie vom Körper verzichtet werden, da die Folie mittels Wasser oder der Reinigungslösung aufgelöst werden kann.

Gemäß einer Weiterbildung des Verfahrens kann die Folie transparent ausgebildet sein. Die Folie kann insbesondere für das menschliche Auge transparent bzw. durchsichtig ausgebildet sein. Es kann eine versatzfreie Verklebung und/oder Positionierung von umlaufenden und/oder mehrteiligen Folien (bzw. Konzepten) umgesetzt werden.

Hierdurch kann die Folie einfacher auf dem Körper an- bzw. aufgebracht und positioniert werden. Es kann zudem eine klare Sicht auf den Körper umgesetzt werden. Bei herkömmlichem Tätowieren wird dies durch eine undurchsichtige Papierstruktur verhindert.

Gemäß einer Weiterbildung des Verfahrens kann die Folie eine Dicke (bzw. Stärke) in einem Bereich von 10 µm bis 30 µm (Mikrometer), insbesondere in einem Bereich von 15 µm bis 25 µm, aufweisen. Vorzugsweise kann die Dicke der Folie 15 µm betragen.

Hierdurch kann eine Folie bereitgestellt werden, die einerseits eine ausreichende Festigkeit zur Durchführung der Körpermodifikation bzw. dem Durchstechen und andererseits eine ausreichende Flexibilität zur Anpassung an den Körper (bzw. dessen Unebenheiten), aufweist.

Darüber hinaus kann durch die gewählte Dicke der Folie sichergestellt werden, dass die mittels eines Tätowierwerkzeugs injizierte Tätowierfarbe aus einem Injizierloch in ein anderes bspw. benachbartes Injizierloch fließt, da die Dicke der Folie auf dem Körper als Barriere für die Tätowierfarbe zwischen (benachbarten) Injizierlöchern im Körper dienen kann. Die Injizierlöcher können mittels des Körpermodifizierungswerkzeugs (bspw. einer Tätowiernadel) ausgebildet bzw. erzeugt werden.

Gemäß einer Weiterbildung des Verfahrens kann die Folie eine Breite in einem Bereich von 26 mm bis 841 mm (Millimeter) aufweisen. Die Folie kann eine Höhe in einem Bereich von 37 mm bis 1189 mm aufweisen. Die Folie kann eine Breite von 210 mm und eine Höhe von 297 mm aufweisen.

Die Folien können in standardisierten Größen, insbesondere von DIN A0 bis einschließlich DIN A10, in dem Verfahren zum Einsatz kommen. Die DIN Normen können sich vorzugsweise auf ihren Stand vom 01. Juli 2024 beziehen.

Hierdurch kann auf verschiedengroße Körpermodifikation und verschiedengroße Körper oder Körperstellen eingegangen werden und die verwendete Folie vollständig genutzt werden, wodurch die Kosten des Verfahrens weiter reduziert werden können.

Gemäß einer Weiterbildung des Verfahrens kann die Folie einen thermoplastischen Kunststoff, insbesondere Polyvinylalkohol, umfassen oder daraus (vollständig) ausgebildet sein.

Hierdurch kann die Bedruckbarkeit, die Transparenz, die Verträglichkeit und/oder die Wasserlöslichkeit der Folie mit einfachen Mitteln umgesetzt werden.

Erfindungsgemäß handelt es sich bei der Körpermodifikation um eine Tätowierung oder Permanent-Makeup.

Hierdurch können verschieden Anwendungen umgesetzt werden, wobei die Qualität und Genauigkeit der Körpermodifikation bei geringem Aufwand sowie geringen Kosten gewährleistet werden kann.

Wenn es sich bei der Körpermodifikation um eine Tätowierung handelt, kann es sich bei der Markierung um ein Tattoo-Motiv handeln. Bei dem Tattoo-Motiv kann es sich um ein Tattoo-Design handeln, welches tätowiert werden soll.

Wenn es sich bei der Körpermodifikation um ein Piercing handelt, kann es sich bei der Markierung um eine Piercing-Sollstelle handeln. Bei der Piercing-Sollstelle kann es sich um eine Stelle handeln, an der das Piercing oder der Schmuck platziert werden soll.

Wenn es sich bei der Körpermodifikation um das Aufbringen von Kosmetik oder Makeup handelt, kann die Markierung einer Kosmetik-Sollstelle oder eine Makeup-Sollstelle entsprechen. Eine Kosmetik-Sollstelle kann einer Stelle entsprechen, auf der die Kosmetik aufgebracht werden soll. Eine Makeup-Sollstelle kann einer Stelle entsprechen, auf der das Makeup aufgebracht werden soll.

Die obige Aufgabe wird weiter durch einen Verbundbogen mit den Merkmalen des Anspruchs 9 gelöst.

Der Verbundbogen umfasst ein Trägerpapier und eine Folie. Die Folie ist zur Durchführung des Verfahrens gemäß obiger Ausführungen ausgebildet. Die Folie ist am Trägerpapier befestigt. Die Folie ist mittels eines an einem Rand des Trägerpapiers angeordneten Klebeabschnitts am Trägerpapier befestigt. Die Folie kann mittels Stanzen, Verschweißungen (bzw. Schweißen) und/oder Perforierungen mit dem Trägerpapier verbunden bzw. am Trägerpapier befestigt sein.

Das Trägerpapier weist eine Grammatur in einem Bereich von 60 g/m² bis 100 g/m² (Gramm pro Quadratmeter), insbesondere 80 g/m² auf.

Hinsichtlich der mit dem Verbundbogen erzielbaren Vorteile wird auf die diesbezüglichen Ausführungen zum Verfahren verwiesen. Zur weiteren Ausgestaltung des Verbundbogens oder seiner Bestandteile können die im Zusammenhang mit dem Verfahren beschriebenen und/oder die nachfolgend noch erläuterten Maßnahmen und Merkmale dienen.

Durch das Trägerpapier kann sichergestellt werden, dass die daran befestigte Folie mittels eines Papiereinzugs eines Tintenstrahldruckers eingezogen und somit durch den Tintenstrahldrucker bedruckt werden kann.

Der Klebeabschnitt ist streifenförmig ausgebildet. Der Klebeabschnitt ist an einem Randabschnitt des Trägerpapiers bzw. der Folie angeordnet.

Das Trägerpapier kann eine rechteckige Form mit zwei langen Seiten und zwei kurzen Seiten umfassen, wobei sich der Randabschnitt an einer kurzen Seite des Trägerpapiers befinden kann. Der Klebeabschnitt kann sich entlang der kurzen Seite erstrecken.

Bei dem Klebeabschnitt kann es sich um eine stoffschlüssige Verbindung zwischen der Folie und dem Trägerpapier handeln.

Die Folie kann markierungsfrei sein oder mindestens eine Markierung aufweisen, die als Vorlage für eine Körpermodifikation dient, wobei die Markierung insbesondere einem Tattoo-Motiv oder Tattoo-Design, einer Piercing-Sollstelle, einer Kosmetik-Sollstelle oder einer Makeup-Sollstelle entsprechen kann. Ein Verbundbogen mit einer markierungsfreien Folie kann mit mindestens einer Markierung bedruckt werden. Insbesondere kann hierbei die Folie bedruckt werden. Dies kann bspw. mittels eines (herkömmlichen) Tintenstrahldruckers umgesetzt werden.

Zur Durchführung des Verfahrens zur Durchführung einer Körpermodifikation kann die Folie vor dem Anbringen (Aufbringen) auf dem Körper vom Trägerpapier entfernt werden. Die Folie vor dem Entfernen vom Trägerpapier mit dem Tattoo-Design oder dem Tattoo-Motiv bspw. mittels eines Tintenstrahldruckers bedruckt werden.

Weitere Merkmale, Einzelheiten und Vorteile der Erfindung ergeben sich aus dem Wortlaut der Ansprüche sowie aus der folgenden Beschreibung von Ausführungsbeispielen anhand der Zeichnungen. Es zeigen schematisch:
- Fig. 1: ein Verfahren zur Durchführung einer Körpermodifikation,
- Fig. 2: eine Schnittansicht gemäß dem Schnitt A-A aus Figur 1,
- Fig. 3: ein Ablaufdiagramm des Verfahrens gemäß Figur 1 und
- Fig. 4: einen Verbundbogen.

In der nachfolgenden Beschreibung sowie in den Figuren tragen sich entsprechende Bauteile und Elemente gleiche Bezugszeichen. Der besseren Übersichtlichkeit wegen sind nicht in allen Figuren sämtliche Bezugszeichen wiedergegeben.

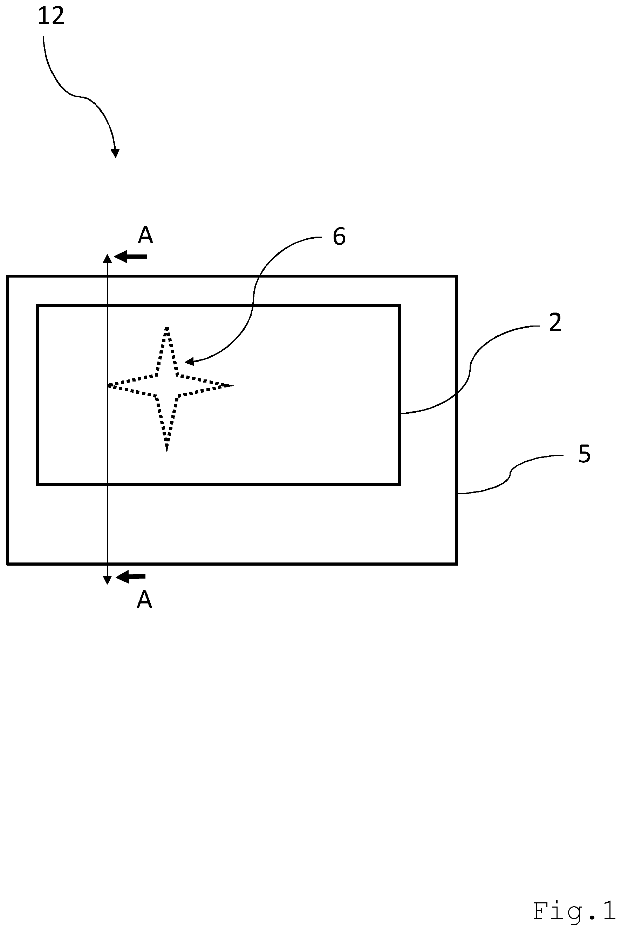

Figur 1 zeigt schematisch ein Verfahren 12 zur Durchführung einer Körpermodifikation.

Figur 1 zeigt eine Folie 2, die eine Markierung 6 umfasst und die auf einem Körper 5 angebracht ist. Die Markierung 6 entspricht vorliegend einem sternenförmigen Tattoo-Design, welches auf den Körper 5, hier die Haut eines Körpers 5, tätowiert werden soll. Ferner ist ein Schnittverlauf A-A dargestellt, der den Körper 5 sowie die Folie 2 im Bereich der Markierung 6 schneidet.

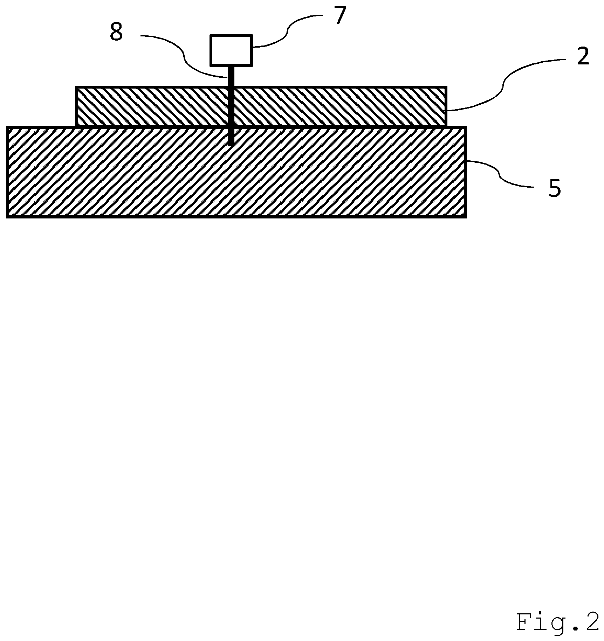

Figur 2 zeigt eine Schnittansicht gemäß dem Schnitt A-A aus Figur 1. Es ist ein Körpermodifikationswerkzeug 7 mit einer Nadel 8 dargestellt, welches die Folie 2 durchsticht. Die Nadel 8 des Körpermodifikationswerkzeugs 7 sticht vorliegend in den Körper 5 durch die Folie 2 hindurch.

Bei dem Körpermodifikationswerkzeug 7 handelt es sich vorliegend um eine Tätowierpistole, während es sich bei der Nadel 8 um eine Tätowiernadel handelt. Mittels der Nadel 8 kann die Folie 2 entlang der Markierung 6 (vgl. Figur 1) mehrfach, sukzessiv durchstochen und in eine Hautschicht des Körpers 5 eingestochen werden, um Tätowierfarbe in den Körper 5 zu injizieren, bis die Tätowierung, entsprechend dem Tattoo-Design, fertiggestellt ist.

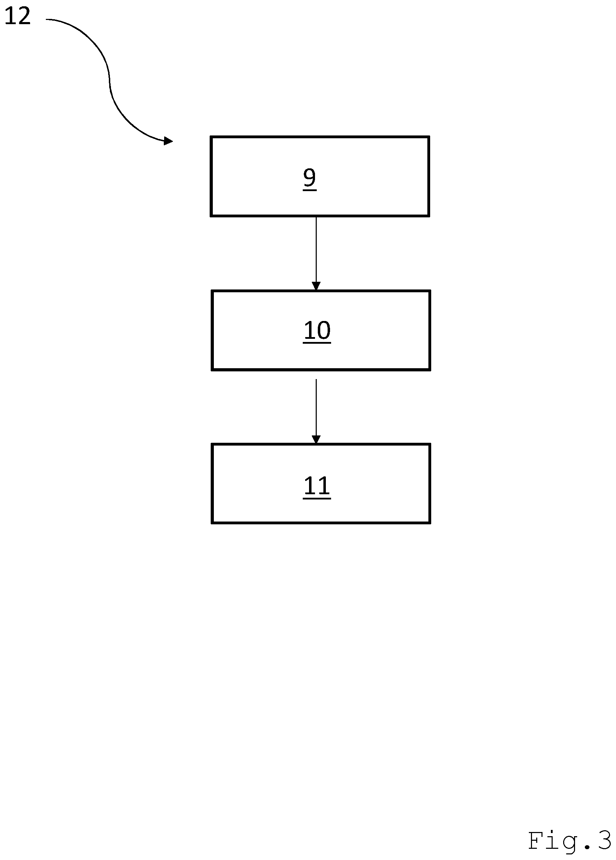

Figur 3 zeigt ein Ablaufdiagramm des Verfahrens 12 gemäß Figur 1. Das Verfahren 12 umfasst die Schritte:
9: Anbringen einer Folie 2 auf einen zu modifizierenden Körper 5, wobei die Folie 2 mindestens eine Markierung 6 zur Durchführung der Körpermodifikation aufweist.

10: Durchführen der Körpermodifikation am Körper 5 entsprechend der Markierung 6, wobei die Folie 2 während der Durchführung der Körpermodifikation an der Markierung 6, insbesondere mittels eines Körpermodifikationswerkzeugs, durchdrungen wird, um den Körper 5 zu modifizieren.

Das Verfahren kann den Schritt umfassen:
11: Auflösen der Folie 2 mittels (kaltem) Wasser und/oder einer Reinigungslösung.

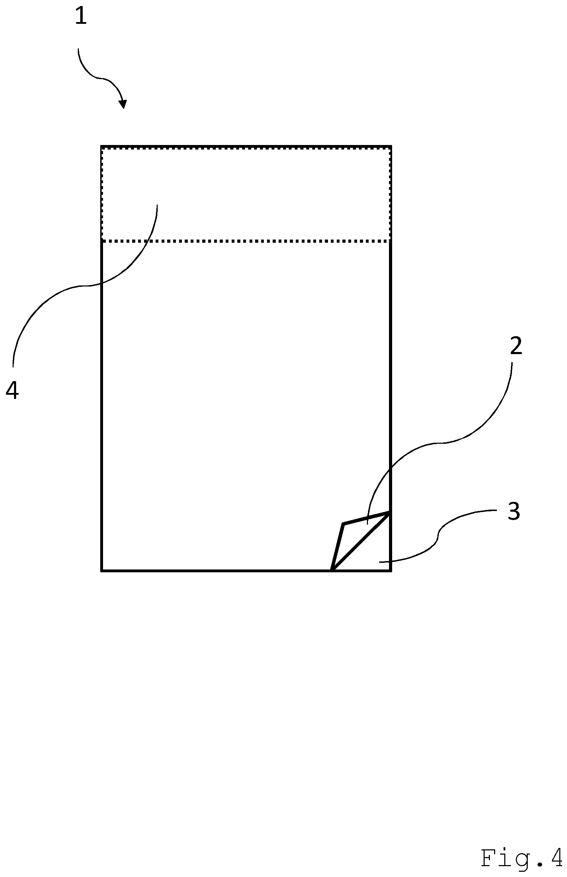

Die Markierung 6 kann auf die Folie 2 aufgedruckt werden, bevor die Folie 2 auf dem Körper 5 aufgebracht wird. Dies kann mittels eines Tintenstrahldrucker umgesetzt werden. Das Bedrucken der Folie 2 mit der Markierung 6 kann auf einem Verbundbogen 1 erfolgen (vgl. Figur 4).

Es kann eine hygienische Reinigung der Körpers 5 an der zu tätowierenden Körperstelle erfolgen, um eine Infektion zu verhindern.

Die Markierung auf der Folie 2 entspricht vorliegend einem Tattoo-Design, das auf den Körper 5 tätowiert werden soll.

Das Anbringen der Folie 2 kann mittels einer temporären Klebeverbindung, insbesondere mittels eines sprüht Klebers, erfolgen.

Die Folie 2 kann wasserlöslich, insbesondere kalt wasserlöslich, ausgebildet sein. Alternativ oder zusätzlich kann die Folie 2 nach der Durchführung der Körpermodifikation mittels Wasser (kaltem Wasser) und/oder einer Reinigungslösung aufgelöst werden.

Die Folie 2 kann für das menschliche Auge transparent ausgebildet sein.

Die Folie 2 kann eine Dicke in einem Bereich von 10 µm bis 30 µm, insbesondere in einem Bereich von 15 µm bis 25 µm, aufweisen.

Die Folie 2 kann eine Breite in einem Bereich von 26 mm bis 841 mm aufweisen. Die Folie 2 kann eine Breite von 210 mm aufweisen. Die Folie 2 kann eine Höhe in einem Bereich von 37 mm bis 1189 mm aufweisen. Die Folie 2 kann eine Höhe von 297 mm aufweisen.

Die Folie 2 kann einen thermoplastischen Kunststoff, insbesondere Polyvinylalkohol, umfassen oder daraus ausgebildet sein.

Bei der Körpermodifikation kann es sich um eine Tätowierung, um ein Piercing oder um das Aufbringen von Kosmetik oder Permanent-Makeup handeln.

Figur 4 zeigt einen Verbundbogen 1. Der Verbundbogen 1 umfasst ein Trägerpapier 3 und eine Folie 2. Die Folie 2 ist zur Durchführung des Verfahrens gemäß obiger Ausführungen eingerichtet. Insbesondere handelt es sich bei der Folie 2 um die in den Figuren 1 und 2 gezeigte Folie 2.

Die Folie 2 ist vorliegend mittels eines Klebeabschnitts 4 am Trägerpapier 3 befestigt. Der Klebeabschnitt 4 ist an einem Rand des Trägerpapiers 3 angeordnet.

Die Folie 2 kann ebenso mittels Stanzen, Verschweißungen (bzw. Schweißen) und/oder Perforierungen mit dem Trägerpapier 3 verbunden bzw. am Trägerpapier 3 befestigt sein.

Das Trägerpapier 3 weist eine Grammatur in einem Bereich von 60 g/m² bis 100 g/m², insbesondere 80 g/m², auf.

### Bezugszeichenliste

- 1: Verbundbogen
- 2: Folie
- 3: Trägerpapier
- 4: Klebeabschnitt
- 5: Körper
- 6: Markierung
- 7: Körpermodifikationswerkzeug
- 8: Nadel
- 9: Verfahrensschritt
- 10: Verfahrensschritt
- 11: Verfahrensschritt
- 12: Verfahren

## Patentansprüche

1. Verfahren (12) zur Durchführung einer Körpermodifikation, umfassend die Schritte:
- (9) Anbringen einer Folie (2) auf einen zu modifizierenden Körper (5), wobei die Folie (2) mindestens eine Markierung (6) zur Durchführung der Körpermodifikation aufweist,
- (10) Durchführen der Körpermodifikation am Körper (5) entsprechend der Markierung (6), wobei die Folie (2) während der Durchführung der Körpermodifikation an der Markierung (6), insbesondere mittels eines Körpermodifikationswerkzeugs, durchstochen wird, um den Körper (5) zu modifizieren,
- Einstechen in den Körper (5), insbesondere mittels des Körpermodifikationswerkzeugs,
- wobei es sich bei der Körpermodifikation um eine Tätowierung oder Permanent-Makeup handelt.

2. Verfahren (12) nach Anspruch 1, **dadurch gekennzeichnet, dass** die Markierung (6) auf die Folie (2), insbesondere mittels eines Tintenstrahldruckers, aufgedruckt wird, bevor die Folie (2) auf dem Körper (5) aufgebracht wird.

3. Verfahren (12) nach Anspruch 1 oder 2, **dadurch gekennzeichnet, dass** das Anbringen der Folie (2) mittels einer temporären Klebeverbindung, insbesondere mittels eines Sprühklebers, erfolgt.

4. Verfahren (12) nach einem der voranstehenden Ansprüche, **dadurch gekennzeichnet, dass** die Folie (2) wasserlöslich, insbesondere kaltwasserlöslich, ausgebildet ist und/oder die Folie (2) nach der Durchführung der Körpermodifikation mittels Wasser und/oder einer Reinigungslösung aufgelöst wird.

5. Verfahren (12) nach einem der voranstehenden Ansprüche, **dadurch gekennzeichnet, dass** die Folie (2) für das menschliche Auge transparent ausgebildet ist.

6. Verfahren (12) nach einem der voranstehenden Ansprüche, **dadurch gekennzeichnet, dass** die Folie (2) eine Dicke in einem Bereich von 10 µm bis 30 µm, insbesondere in einem Bereich von 15 µm bis 25 µm, aufweist.

7. Verfahren (12) nach einem der voranstehenden Ansprüche, **dadurch gekennzeichnet, dass** die Folie (2) eine Breite in einem Bereich von 26 mm bis 841 mm, insbesondere 210 mm, und eine Höhe in einem Bereich von 37 mm bis 1189 mm, insbesondere 297 mm, aufweist.

8. Verfahren (12) nach einem der voranstehenden Ansprüche, **dadurch gekennzeichnet, dass** die Folie (2) einen thermoplastischen Kunststoff, insbesondere Polyvinylalkohol, umfasst oder daraus ausgebildet ist.

9. Verbundbogen (1), umfassend ein Trägerpapier (3) und eine Folie (2), wobei die Folie (2) zur Durchführung des Verfahrens (12) nach einem der voranstehenden Ansprüche ausgebildet ist, wobei die Folie (2) mittels eines an einem Rand des Trägerpapiers (3) angeordneten Klebeabschnitts (4) am Trägerpapier (3) befestigt ist, wobei der Klebeabschnitt (4) streifenförmig ausgebildet ist, wobei das Trägerpapier (3) eine Grammatur in einem Bereich von 60 g/m² bis 100 g/m², insbesondere 80 g/m², aufweist.

## Claims

1. Method (12) for performing a body modification, comprising the steps of:
- (9) applying a film (2) to a body (5) to be modified, wherein the film (2) has at least one marking (6) for carrying out the body modification,
- (10) performing the body modification on the body (5) according to the marking (6), wherein the film (2) is punctured at the marking (6) during the performance of the body modification, in particular by means of a body modification tool, in order to modify the body (5),
- piercing the body (5), in particular using the body modification tool,
- wherein the body modification is a tattoo or permanent makeup.

2. Method (12) according to claim 1, **characterized in that** the marking (6) is printed onto the film (2), in particular by means of an inkjet printer, before the film (2) is applied to the body (5).

3. Method (12) according to claim 1 or 2, **characterized in that** applying the film (2) is carried out by means of a temporary adhesive bond, in particular by means of a spray adhesive.

4. Method (12) according to any of the preceding claims,
**characterized in that** the film (2) is water-soluble, in particular cold-water-soluble, and/or the film (2) is dissolved by means of water and/or a cleaning solution after the body modification has been performed.

5. Method (12) according to any of the preceding claims,
**characterized in that** that the film (2) is transparent to the human eye.

6. Method (12) according to any of the preceding claims,
**characterized in that** the film (2) has a thickness in a range of 10 µm to 30 µm, in particular in a range of 15 µm to 25 µm.

7. Method (12) according to any of the preceding claims, **characterized in that** the film (2) has a width in a range of 26 mm to 841 mm, in particular 210 mm, and a height in a range of 37 mm to 1189 mm, in particular 297 mm.

8. Method (12) according to any of the preceding claims,
**characterized in that** the film (2) comprises or is formed from a thermoplastic plastics material, in particular polyvinyl alcohol.

9. Composite sheet (1) comprising a backing paper (3) and a film (2), wherein the film (2) is designed to carry out the method (12) according to any of the preceding claims, wherein the film (2) is attached to the backing paper (3) by means of an adhesive portion (4) arranged at an edge of the backing paper (3), wherein the adhesive portion (4) is strip-shaped, wherein the backing paper (3) has a basis weight in a range of 60 g/m² up to 100 g/m², in particular 80 g/m².

## Revendications

1. Procédé (12) pour la réalisation d'une modification corporelle, comprenant les étapes :
- (9) Pose d'une feuille (2) sur un corps (5) à modifier, la feuille (2) comportant au moins un repère (6) pour la réalisation de la modification corporelle,
- (10) Réalisation de la modification corporelle sur le corps (5) conformément au repère (6), la feuille (2) étant, pendant la réalisation de la modification corporelle, percée au niveau du repère (6), notamment au moyen d'un outil de modification corporelle, afin de modifier le corps (5),
- Piqûre dans le corps (5), notamment au moyen de l'outil de modification corporelle,
- la modification corporelle consistant en un tatouage ou en un maquillage permanent.

2. Procédé (12) selon la revendication 1, **caractérisé en ce que** le repère (6) est imprimé sur la feuille (2), notamment au moyen d'une imprimante à jet d'encre, avant que la feuille (2) ne soit appliquée sur le corps (5).

3. Procédé (12) selon la revendication 1 ou 2, **caractérisé en ce que** la pose de la feuille (2) s'effectue par une liaison adhésive temporaire, en particulier par un adhésif pulvérisable.

4. Procédé (12) selon l'une quelconque des revendications précédentes, **caractérisé en ce que** la feuille (2) est hydrosoluble, en particulier soluble à l'eau froide, et/ou que la feuille (2) est dissoute après la réalisation de la modification corporelle au moyen d'eau et/ou d'une solution de nettoyage.

5. Procédé (12) selon l'une quelconque des revendications précédentes, **caractérisé en ce que** la feuille (2) est transparente à l'œil nu.

6. Procédé (12) selon l'une quelconque des revendications précédentes, **caractérisé en ce que** la feuille (2) a une épaisseur comprise entre 10 µm et 30 µm, en particulier entre 15 µm et 25 µm.

7. Procédé (12) selon l'une quelconque des revendications précédentes, **caractérisé en ce que** la feuille (2) présente une largeur dans une plage de 26 mm à 841 mm, notamment 210 mm, et une hauteur dans une plage de 37 mm à 1189 mm, notamment 297 mm.

8. Procédé (12) selon l'une quelconque des revendications précédentes, **caractérisé en ce que** la feuille (2) comprend un matériau thermoplastique, notamment l'alcool polyvinylique, ou est formée à partir de celui-ci.

9. Feuillet composite (1), comprenant un papier support (3) et une feuille (2), la feuille (2) étant conçue pour la réalisation du procédé (12) selon l'une quelconque des revendications précédentes, la feuille (2) étant fixée au papier support (3) au moyen d'une section adhésive (4) disposée à un bord du papier support (3), ladite section adhésive (4) étant formée en bande, le papier support (3) présentant un grammage dans une plage de 60 g/m2 à 100 g/m2, notamment 80 g/m2.
